Europäisches Patentamt

European Patent Office

Office européen des brevets

(11) Publication number: **0 310 288 A2**

# EUROPEAN PATENT APPLICATION

(21) Application number: **88308741.3**

(22) Date of filing: **21.09.88**

(51) Int. Cl.⁴: **A61K 37/38**

(30) Priority: **01.10.87 AU 4664/87**

(43) Date of publication of application:
**05.04.89 Bulletin 89/14**

(84) Designated Contracting States:
**AT BE CH DE ES FR GB GR IT LI LU NL SE**

(71) Applicant: **Bunge (Australia) Proprietary Limited**
**6th Floor 616 St. Kilda Road**
**Melbourne Victoria 3004(AU)**

(72) Inventor: **Brandon Malcolm Roy**
**14 Castella Street**
**Ivanhoe, Victoria(AU)**

(74) Representative: **Harding, Richard Patrick et al**
**Arthur R. Davies & Co. 27 Imperial Square**
**Cheltenham GL50 1RQ(GB)**

(54) **Improved ovulation treatment.**

(57) In order to increase the number of ova produced during ovulation in a female animal, an ovine follicle stimulating hormone is administered to the animal. Where the animal is a bovine animal the hormone is administered in a total amount of from approximately 20 to 60 u in at least three substantially equal doses at at least three regular intervals during a period of one day, depending upon the size of the animal. Where the animal is an ovine or caprine animal the hormone is administered in an amount of from approximately 20 to 40 u in a single dose.

EP 0 310 288 A2

## Improved Ovulation Treatment

This invention relates to a method of regulating the reproductive functions of animals, in particular female animals, and to veterinary compositions for use in such a method.

It is known in the prior art to regulate reproductive functions in female animals in a variety of ways. Artificial and natural products such as prostaglandins, pregnant mare serum gonadotrophin, melatonins and the like have been proposed for regulation of reproduction in animals. However, such treatments have proved of limited value in inducing or increasing ovulation in female animals.

One treatment known in the prior art includes the treatment of animals with a product incorporating porcine follicle-stimulating hormone (P-FSH). While such treatments have proved to be effective, it has been found necessary to utilise relatively high dosage rates which are therefore expensive and may lead to complications, due to over-stimulation, including ovarian damage, generalised oedema, and adhesions and may impair subsequent fertility. Moreover, difficulties have been found in the quality of embryos subsequently produced and a high proportion of unviable embryos has been found to be common.

It is therefore an object of the present invention to overcome, or at least alleviate, one or more of the difficulties related to the prior art.

Accordingly, in accordance with a first aspect of the present invention there is provided a method of increasing the number of ova produced during ovulation in female animals which method includes administering to the animal an ovine follicle stimulating hormone (as hereinbefore defined) wherein when the animal is a bovine animal the hormone is administered in an amount of from approximately 20 to 60 u in at least three substantially equal doses at at least three regular intervals during a period of 1 day, dependant upon the size of the animal; and

when the animal is an ovine or caprine animal the hormone is administered in an amount of from approximately 20 to 40 u in a single dose.

The amount of ovine follicle stimulating hormone is measured as unit potency of ovine FSH, "u". This is the standard established by the United States National Institute of Health, Technical Report Number 156 of the Pituitary Hormones and Antisera Center.

Preferably where the animals to be treated are sheep or goats the oFSH may be administered in amounts from approximately 20 to 30 u in a single dose.

It has surprisingly been found that administration in the simplified form of a simple dose of oFSH on a one day produces excellent superovulation levels in sheep and goats. This is a substantial improvement over the prior art since the simplicity for the user in administering a single dose cannot be over estimated. This is a surprising result and could not be duplicated with other forms of follicle-stimulating hormone such as P-FSH.

As stated where the animals to be treated are cattle, the oFSH may be administered in three equal doses at substantially equal intervals during a single day. Whilst this dosage regimen is not quite as convenient as a single dose, it still provides a substantial improvement over the prior art.

As stated above, the dose rate for cattle will vary with the size of the animal. The total daily dose rate may be approximately 0.06 to 0.24 u/kg live weight, more preferably approximately 0.09 to 0.15 u/kg live weight. Thus where three equal doses are administered in a single day, each dose may be approximately 0.02 to 0.08 u/kg live weight.

By the term "ovine follicle-stimulating hormone" as used herein in the description and claims we mean a natural or synthetic ovine follicle-stimulating hormone, a derivative thereof or bioprecursor therefor.

By the term "bioprecursor" as used herein in the description and claims we mean any compound which exhibits ovine follicle-stimulating hormone-like activity in animals.

A follicle-stimulating hormone isolated from a sexually immature animal is preferred.

The method of treating the female animal may be of any suitable types. The treatment may be oral, by injection, by implant or the like. Accordingly, the ovine follicle stimulating hormone may be provided in a unit dosage form. An oral form, an injectable form or an implant form may be used. The follicle-stimulating hormone may be in a lyophilised form.

The follicle-stimulating hormone may be provided from natural or synthetic sources including the cloning of the gene coding for a follicle-stimulating hormone which is a polypeptide and its expression in prokaryotic and eukaryotic organisms, or a product thereof may also be used. The follicle-stimulating hormone may be extracted from the pituitary gland of sheep. The pituitary glands of lambs are preferred. Ovine follicle stimulating hormone isolated from the pituitary glands of lambs has been found to be surprisingly more effective than other forms.

2

It has been surprisingly found that ovine follicle-stimulating hormone provides an improved yield of ova relative to other treatments including treatment with P-FSH. An increased yield of approximately 50% or greater may be provided. Moreover, it has been found that embryos produced from ova induced by ovine follicle-stimulating hormone are of improved quality relative to prior art treatments and damage to the ovaries of the treated animals is surprisingly reduced. The embryos so produced are characterised by an increased viability.

The amount of ovine follicle-stimulating hormone to be used will vary with the species of animal to be treated and the reproductive function to be achieved.

The selection of dose rates is also dependent on the source of follicle stimulating hormone. The range of dose rates stated above relates to FSH isolated from natural sources including isolation from harvested pituitary glands of animals. Such "natural" FSH may include a high level of impurities. FSH having a higher purity for example where formed via a synthetic route may be administered at reduced dose rates.

The single dose may be administered via intramuscular injection. The pre-pubescent animals may be treated at reducing dosage rates over a period of approximately 1 to 5 days.

In a preferred form, the method increasing ovulation may include the preliminary step of treating the animal with an effective amount of a synchronising agent. The synchronising agent may be selected from any of the agents known per se. The agent may be a progestagen, prostaglandin, prostaglandin analogue or the like. Synchronising agents sold under the trade designation Chronogest (available Intervet (Australia) and "Repromap" (available from Upjohn Pty. Ltd.) intravaginal sponges have been found to be suitable in sheep and goats. The synchronising agent sold under the trade designation Estrumate (available from ICI Australia Ltd.) has been found to be suitable for cattle.

The preliminary treatment with the synchronising agent or the like may be undertaken at a preselected interval of time prior to the initiation of treatment with the ruminant follicle-stimulating hormone. The treatment with synchronising agent may be undertaken approximately 8 to 15 days prior to FSH treatment.

Preferably approximately 250 ug to approximately 100 mg of a synchronising agent is administered to a pre-pubescent animal approximately 8 to 15 days prior to initiation of ovine follicle-stimulating hormone treatment. The treatment with synchronising agent may be repeated at a suitable interval if necessary .

Preferably approximately 500 ug to 1000 ug of synchronising agent is used in the treatment of cattle via an injectable synchronising agent such as "Estrumate".

Preferably approximately 25 to 75 mg of synchronising agent is used in the treatment of sheep and goats via a synchronising agent of the intravaginal-sponge type such as Repromap or Chronogest.

In a more preferred form, the method of increasing ovulation may include the further step of treating the animal with an effective amount of a luteinizing agent during or after the ovine FSH treatment. The luteinizing agent may be a prostaglandin or prostaglandin analogue. The luteinizing agent may be the same as, or different to the synchronising agent previously described.

Preferably the luteinizing agent is a prostaglandin or prostaglandin analogue and is administered in an amount of approximately 250 ug to approximately 1000 ug approximately 1 to 3 days after the initiation of ruminant follicle-stimulating hormone treatment.

In a further preferred form the method of inducing or increasing ovulation may include the further step of administering to the animal an effective amount of gonadotrophin or derivative thereof.

It has been found that some animals in some trials of the order of 20% do not respond to the ovine FSH treatment done. It has now been found that an additional treatment with a relatively small amount of a gonadotrophin or derivative thereof results in a substantial reduction of the number of animals who are not responding.

A pregnant mare serum gonadotrophin (PMSG) may be used. A pregnant mare serum gonadotrophin of the type described in Australian provisional patent application PH 8482/86 has been found to be suitable (the entire disclosure of which is incorporated herein by reference). More preferably the monoclonal antibody derived therefrom disclosed therein may be used. PMSG is also referred to as equine chorionic gonadotrophin (eCG).

The gonadotrophin or derivative thereof may be administered in amounts of from approximately 100 i.u to 1000 i.u. and preferably 500 i.u.

The gonadotrophin treatment may be administered at any time during or before the ovine FSH treatment. Preferably the gonadotrophin treatment may be undertaken on the first day of the ovine FSH treatment.

In a further aspect of the present invention there is provided a veterinary composition including an effective amount of an ovine follicle-stimulating hormone in a lyophilised form. The veterinary composition may further include a veterinarily acceptable carrier or excipient. The carrier or excipient may be a solvent for the follicle stimulating hormone or derivative. An aqueous solvent may be used. A physiologically

3

acceptable saline buffered solvent may be used. The veterinary composition may be provided in an injectable form.

In a preferred form of this aspect of the present invention the veterinary composition may further include an effective amount of a gonadotrophin or gonadotrophin analogue.

In a preferred form the present invention provides a kit of parts including a supply of ovine follicle-stimulating hormone in a lyophilised form in a suitable container.

The kit of parts may further include a supply of a synchronising agent in a suitable container. The ovine follicle-stimulating hormone, gonadotrophin, and the synchronising agent where present, may be provided in an injectable form.

The present invention will now be more fully described with reference to the following examples. It should be understood however, that the description following is illustrative only and should not be taken in any way as a restriction on the generality of the invention described above.

In the examples following, the abbreviation "i.m." refers to intra-muscular injection.

## EXAMPLE 1

The ovine follicle-stimulating hormone (oFSH) was prepared in the following way:

Frozen whole lamb pituitaries were ground and extracted overnight with distilled water, pH 5.5. The resulting FSH-rich extract was fractionated successively with 1.80M ammonium sulphate, pH 4.0 to remove inert proteins, and the FSH precipitated with 3.0 M ammonium sulphate, pH 4.0.

The FSH preparation was neutralised with NaOH, dialysed against distilled water, and lyophilized. All steps of this procedure were carried out in a cold room at 5° C.

## EXAMPLE 2

Field trials were conducted on sheep utilising ovine follicle-stimulating hormone (oFSH). The dosage of oFSH was initially measured in mg of pituitary prepared material. For all studies whose mg of oFSH are specified, 1 mg of oFSH is equivalent to 2 units of oFSH by reference to NIH standard. The sheep were treated utilising the following treatment regimen:

### Sheep Regimen for FSH

8 injections over 4 days (a.m.,p.m.) in decreasing amounts in the ratio of 4:3:2:1

For example

8 mg as follows:

        Day 1
- a.m.    1.6
- p.m.    1.6
        Day 2
- a.m.    1.2
- p.m.    1.2
        Day 3
- a.m.    0.8
- p.m.    0.8
        Day 4
- a.m.    0.4
- p.m.    0.4

Treatment Schedule

Day 1 - REPROMAP * sponge inserted
Day 10 - day 1 of injection of FSH a.m./p.m.
Day 11 - day 2     FSH a.m./p.m.
Day 12 - sponge withdrawal day 3 FSH a.m./p.m.
Day 13 - day 4 FSH a.m./p.m.

The results of three field trials including a comparison with ewes treated with the commercially available P-FSH (available from ESSEX/Heriot, Melbourne) are set out in Tables 1, 2 and 3.

TABLE 1

| Trial 1 Random Bred Merinos | | | | Ovulation Rate* | |
|---|---|---|---|---|---|
| | Dose Total mg | Ewes treated | Ovulated following treatment | Mean | Range |
| Ovine FSH | 8 | 8 | 7 | 4.25 | 1-19 |
| | 4 | 7 | 7 | 1.6 | 1-3 |
| | 2 | 8 | 6 | 1.2 | 1-2 |
| Commercial Product P-FSH | 8 | 7 | 7 | 2.4 | 1-5 |
| | 4 | 6 | 5 | 1.2 | 1-2 |
| * of those ovulating | | | | | |

TABLE 2

| Trial 2 Random Bred Merinos | | | | Ovulation Rate | |
|---|---|---|---|---|---|
| | Dose Total mg | Ewes Treated | Ovulated following treatment | Mean | Range |
| Ovine FSH | 8 | 8 | 6 | 5.83 | 1-14 |
| | 4 | 9 | 9 | 1.33 | 1-2 |
| | 2 | 9 | 9 | 1.67 | 1-3 |
| | 0 | 8 | 8 | 1.25 | 1-2 |
| P-FSH | 8 | 8 | 7 | 3.71 | 1-11 |
| | 4 | 8 | 8 | 1.38 | 1-2 |
| | 0 | 8 | 8 | 1.25 | 1-2 |
| * of those ovulating | | | | | |

*Intravaginal    sponge    (60  mg  of  Medroxyprogesterone      acetate)

## TABLE 3

### Trial 3 First Cross Ewes (Border Leicester x Merino)

| | Dose Total mg | Ewes Treated | Ovulated following treatment | Ovulation Rate* Mean | Range |
|---|---|---|---|---|---|
| Ovine FSH | 12+ | 12 | 11 | 10.2 | 2-23 |
| P-FSH | 18+ | 6 | 6 | 4.3 | 1-6 |

*of those ovulating

+ In this field trial the FSH was administered AM, PM over 3 days starting at Day 10 after sponge insertion.
The amounts (mg) of oFSH injected were 3,3,2,2,1,1
of P-FSH injected were 4,4,3,3,2,2

## EXAMPLE 3

Excellent results were obtained with oFSH in sheep in contrast to P-FSH. However up to 20% of sheep failed to ovulate using oFSH or P-FSH. This problem was solved by using a small amount of PMSG in conjunction with FSH treatment, resulting in up to 100% of sheep ovulating. The PMSG was preferably that described in Australian provisional patent application No. PH 8482/86 or less preferably the commercially available product Folligon (Intervet, Melbourne). The oFSH proved to be far superior to that of P-FSH when used in conjunction with PMSG.

Field trials were conducted on sheep utilising ovine follicle stimulating hormone (oFSH) and PMSG.

The sheep were treated utilising the following treatment regimen:

### Sheep Regimen for O-FSH

Injections over 3 days (a.m., p.m.) in decreasing amounts in the ratio of 3:2:1
12 mg as follows:

Day 1
- a.m.   3
- p.m.   3

Day 2
- a.m.   2
- p.m.   2

Day 3
- a.m.   1
- p.m.   1

Treatment Schedule

Day 1 - Chronogest * sponge inserted (30mg.)
Day 10 - 500 i.u. PMSG day 1 of injection FSH am/pm
Day 11 - day 2 of injection FSH am/pm
Day 12 - sponge withdrawal day 3 FSH am/pm
Day 13 - Endoscopic artificial insemination PM
Day 19/20 - Flushing of embryos

TABLE 5

| Trial 4 | | | | | |
|---|---|---|---|---|---|
| 50kg Merino Ewes | | | | | |
| | Dose(mg) Total | Ewes treated | Ovulated following treatment | Ovulation Rate* | |
| | | | | Mean | Range |
| Ovine FSH 500 i.u. PMSG | 12 | 16 | 16 | 15.1 | 2-32 |
| 50 kg Merino Ewes | | | | | |
| P-FSH 500 i.u. PMSG | 12 | 25 | 20 | 9.0 | 1-25 |
| 50 kg Comeback Ewes | | | | | |
| Ovine FSH 500 i.u. PMSG | 12 | 11 | 11 | 13.6 | 6-19 |
| 60 kg Southdown Ewes | | | | | |
| Ovine FSH 500 i.u. PMSG | 12 | 11 | 11 | 10.2 | 4-21 |
| * of those ovulating | | | | | |

Observations on Trial 4

1. Fertilisation rate using fresh semen 95% with O-FSH.
2. In all cases where oFSH was used the anatomy of the ovaries was unaltered, whereas where P-FSH was used ovaries were distorted and normal in anatomical appearance.
3. Using P-FSH still 20% of ewes did not ovulate even though a small amount of PMSG used. With O-FSH 100% of sheep ovulated regardless of whether Merino or British-type breeds.
4. Of the Merino sheep treated 50% more embryos using oFSH v P-FSH.
5. Different breeds give different responses. Using P-FSH especially in British-type breeds very poor responses were observed.

* Intravaginal    sponge    (30mg    flugestone    acetate)

TABLE 6

| Trial 5 | | | | | |
|---|---|---|---|---|---|
| 50 kg COMEBACK EWES | | | | | |
| | Dose(mg) Total | Ewes Treated | Ovulated following treatment | Ovulation Rate* | |
| | | | | Mean | Range |
| Ovine FSH 500 i.u. PMSG | 12 15 + | 10 8 | 10 8 | 13.6 15.0 | 6-19 5-23 |
| 60 kg SOUTHDOWN EWES | | | | | |
| Ovine FSH 500 i.u. PMSG | 12 15 | 11 12 | 11 12 | 10.2 11.4 | 4-21 6-20 |

* of those ovulating
+ The amounts of oFSH injected were 3,3,2.5,2.5,2,2

Observations on Trial 5

1. Increasing the dose of oFSH made slight improvement in the ovulation rates of both Merino type and English type sheep.
2. Anatomy of ovaries still fine

EXAMPLE 4

Field trials were conducted on goats utilising ovine follicle-stimulating hormone (oFSH). The goats were treated utilising the following treatment regimen:

Goat Regimen for O-FSH

Injections over 3 days (am/pm) in decreasing amounts in the ratio of 3:2:1
12 mg as follows
        Day 1
- am    3
- pm    3
        Day 2
- am    2
- pm    2
        Day 3
- am    1
- pm    1

## Treatment Schedule

Day 1 - Chronogest Sponge inserted (40mg)
Day 16 -
Group 6a: no PMSG     day 1 of injection
Group 6b: 400 i.u. PMSG FSH am/pm

Day 17 - day 2 of injection FSH am/pm
Day 18 - Sponge withdrawal day 3 FSH am/pm
Day 20 - Endoscopic artificial insemination or natural mating
Day 26 - Flushing of embryos.

TABLE 7

| Trial 6a | | | | | |
|---|---|---|---|---|---|
| | Dose(mg) Total | Ewes Treated | Ovulated following treatment | Ovulation Rate* | |
| | | | | Mean | Range |
| Feral Goats | | | | | |
| Ovine FSH | 12 | 21 | 14 | 12.7 | 1-33 |
| Saline (Controls) | 0 | 18 | 14 | 2.0 | 1-3 |
| Trial 6b | | | | | |
| Angora Goats | | | | | |
| Ovine FSH 400 i.u. PMSG | 12 | 9 | 9 | 10.2 | 4-20 |

* of those ovulating

In goats of similar weight and age using 15 mg P-FSH and 400 i.u. PMSG other researchers have only obtained from 3 to 8 ovulations.

## Observations on Trials 6a & 6b

1. Use of PMSG/oFSH results in 100% ovulation rate for goats.
2. Anatomy of ovaries unchanged - a significant problem with P-FSH.
3. 95% fertilisation rate using fresh semen with oFSH.

## EXAMPLE 5

Field trials were conducted on cattle utilising ovine follicle-stimulating hormone (oFSH). The cattle were treated with oFSH in 8 injections over 4 days (am, pm) in decreasing amounts in the ratio of 4:3:2:1.

## Treatment Schedule

The oestrous cycles were synchronised by two injections 500 ug, intramuscular of a prostaglandin analogue (Estrumate, ICI) at 11 day intervals. On day 8-10 of the subsequent cycle the animals commenced

9

a 4 day FSH treatment (2 subcutaneous injections per day at 0900 and 1600 hr) with the total dose of FSH being given in a decreasing ratio of 4:3:2:1 over the 4 days. The prostaglandin analogue was again injected on the morning of the third day of FSH injection.

The ovulation rate was assessed by laproscopy under Barbiturate anaesthesia 4-6 days after oestrous or by non-surgical flushing of embryos.

TABLE 8

| Trial 7 | | | | | |
|---|---|---|---|---|---|
| Field trial conducted on nulliparous Hereford Heifers aged 3 years with superovulation induced by O-FSH and P-FSH. | | | | | |
| | Dose Total(mg) | Cattle Treated | Ovulated Following Treatment | Ovulation Rate | |
| | | | | Mean | Range |
| Ovine FSH | 0 | 6 | 6 | 1.0 | 1-1 |
| | 10 | 6 | 6 | 2.3 | 1-4 |
| | 30 | 7 | 7 | 13.7 | 10-19 |
| P-FSH | 0 | 6 | 6 | 1.0 | 1-1 |
| | 10 | 6 | 6 | 1.2 | 1-2 |
| | 30 | 7 | 7 | 7.9 | 1-19 |

Observations on Trial 7

1) Excellent superovulatory response to 30 mg oFSH
2) Very uniform ovulatory response with no ovarian over-stimulation using oFSH. However with P-FSH both were a problem.

A field trial was conducted to illustrate the method of inducing ovulation in pre-pubescent animals according to the present invention.

Treatment Protocol

(a) 2 x Estrumate injections 11 days apart
(b) Begin oFSH injections (twice daily for 4 days) 8 days after oestrus
(c) Give Estrumate again on morning of the third day of oFSH treatment
(d) Examine ovulations by laproscope 7 days after oestrus or fertilized embryos by non-surgical flushing.

Trial 8

Field trials conducted on pre-pubescent female cattle of 10 months of age on the Potency of Ovine FSH

## TABLE 9A

| | Dose mg Total | Cattle Treated | Ovulated Following treatment | Ovulation Rate Mean | Range |
|---|---|---|---|---|---|
| | | | **Hereford Cattle** | | |
| oFSH | 8mg | 8 | 8 | 6.9 | 1-14 |

## TABLE 9B

### Dexter Cattle

| | Dose mg Total | Cattle Treated | Ovulated Following treatment | Ovulation Rate Mean | Range |
|---|---|---|---|---|---|
| oFSH | 20mg | 2 | 2 | 14.6 | 13-16 |

Observations on Trial 8

Similar results with young cattle have not been achieved by other researchers using P-FSH.

EXAMPLE 6

Preparation of Slow-Release Composition

An experiment was conducted with a slow-release delivery composition.

Phosphatidyl choline (L- -Lecithin) from frozen egg (10% w/v in water) and cholesterol (1% w/v) were placed into a test tube and the mixture sonicated. (1 hr, 40 min) until a homogeneous mixture was achieved. This mixture was then aliquoted into a freeze-dried preparation of crude FSH and buffer salts (phosphate buffered saline) prior to administration.

2 ml of slow-release delivery composition was added to the lyophilised ovine FSH-buffer salts mixture immediately prior to use.

EXAMPLE 7

Field trials were conducted on sheep and goats utilising a veterinary composition including ovine folliclestimulating hormone (oFSH) including the slow-release composition described in Example 6. The sheep and goats were treated utilising the following treatment regimen.

SHEEP

Treatment Protocol

Day 1 - Chronogest sponge * inserted (30 mg)
Day 2 - 500 iu PMSG day 1 of oFSH injection
Day 12 - sponge withdrawal
Day 13 - endoscopic artificial insemination PM with fresh semen
Day 19/20 - Flushing of embryos

Following treatments undertaken with oFSH-12 mg total given in each treatment

Group 1 (Sheep)

One single Im injection of oFSH given 48 hours prior to sponge withdrawal.

Group 2 (Sheep)

One single im injection of "oFSH and slow release vehicle" given 48 hours prior to sponge withdrawal.

Group 3 (Sheep)

Injections over 3 days (am/pm) in decreasing amounts in the ratio of 3:2:1, starting 48 hours prior to sponge withdrawal
12 mg (day 1 - 3,3, day 2 - 2,2, day 3 - 1,1)


GOATS



Treatment Protocol


Day 1 - Chronogest sponge * inserted (40mg)
Day 2 - 400 iu PMSG day 1 of oFSH injection
Day 18 - Sponge withdrawal
Day 20 - Endoscopic artificial insemination or natural mating
Day 26 - Flushing of embryos


Group 4 (Goats)

One single im injection of oFSH given 48 hours prior to sponge withdrawal.


RESULTS




* Intravaginal sponge (30 mg Flugestone acetate)

* Intravaginal sponge (40 mg flugestone acetate)

Group 1 - 8 Sheep Adult Comeback Ewes

Live weight - 50 kg
Summary of fertilised embryos collected = 11.5 (range 5-17)


Group 2 - 8 Sheep Adult Comeback Ewes

Live weight - 50 kg
Summary of fertilised embryos collected = 10.7 (range 6-16)


Group 3 - 8 Sheep Adult Comeback Ewes

Live weight - 50 kg
Summary of fertilised embryos collected = 13.6 (range 6-19)


## RESULTS


Group 4 - 8 Goats Adult Angora Does

Live weight - 35 kg
Summary of fertilised embryos collected - 10.1 (range 8 - 16)

Results in these trials were slightly lower than those obtained in other trials. This can be attributed to those trials being held in mid-winter when ovulation is usually difficult to induce in sheep and goats. The other trials were conducted in the natural breeding season.


## EXAMPLE 8


Field trials were conducted on cattle utilising a veterinary composition including ovine follicle-stimulating hormone (oFSH). The cattle were treated utilising the following treatment regimen.


CATTLE - New OFSH injection protocol


Treatment Protocol


(a) 2 x PG (Estrumate) injections 11 days apart
(b) Begin FSH injections 10 days after last injection of Estrumate.


Following treatments undertaken with "oFSH" - 8 mg total given in each treatment


Group 1

8 injections over 4 days (day 1, 2, 3, 4) in ratio of 4:3:2:1 AM/PM Starting on "Day 1" (relates to 10 days 40 after 2nd injection of Estrumate)


13

Group 2

Three equal injections im of 2.7 mg oFSH (total 8mg) on "Day 2" at 9 am, 1 pm and 5 pm (i.e. over 8 hours)

Group 3

Single injection im of 8mg oFSH on Day 2 at 9 am.

Group 4

Control Group given saline AM/PM on days 1, 2, 3, 4.
* All cattle given 3rd injection of PE (Estrumate 2ml, im) at "9 am on Day 3".

RESULTS

Group 1:

6 cattle
Live weight - 258 kg
Summary of ovulation rates = 14.3 ± 2.4 Range (8-24)

Group 2:

5 cattle
Live weight - 278 kg
Summary of ovulation rates = 11.0 ± 2.1 Range (6-15)

Group 3:

6 cattle
Live weight - 289 kg
Summary of ovulation rates = 3.8 ± 1.8 Range (1-12)

Group 4:

6 cattle
Live weight - 282 kg
Summary of ovulation rates = 1.00 ± 00 Range (1-1)

No sign of overstimulation in any of the cows treated with oFSH.

EXAMPLE 9

Additional trials undertaken in sheep using the simplified, standardised technique of a single intramuscular injection of 25 units of oFSH dissolved in physiologicaL saline.

14

Treatment Protocol

Day 1 - Chronogest sponge * inserted (30mg)
Day 2 - 500 iu PMSG and single intramuscular injection of 25 units of oFSH
Day 12 - Sponge withdrawal
Day 13 - Endoscopic artificial insemination PM with fresh or frozen semen and/or natural mating
Day 19/20 - Flushing of embryos

MERINO EWES

Location : Hamilton, Victoria.
Age : 3 year
General Condition : good condition, weight 50 kg
Study Design : The treated ewes were run in a flock of 20 untreated sheep on improved pasture

TABLE 10

| Number of Embryos Harvested | | | | | |
|---|---|---|---|---|---|
| Ewe ID | oFSH batch 19 | Ewe ID | oFSH Batch 19 | Ewe ID | oFSH batch 19 |
| 001 | 12 | 002 | 16 | 003 | 32 |
| 004 | 17 | 005 | 14 | 006 | 14 |
| Mean | 17.5 | | | | |
| SD | 7.3˙ | | | | |

MERINO EWES

Location : Hamilton, Victoria.
Age : 4 year
General Condition : heavy condition, weight 60 kg
Study Design : The treated ewes were run in a flock of 10 untreated sheep on improved pasture

TABLE 11

| Number of Embryos Harvested | | | | | |
|---|---|---|---|---|---|
| Ewe ID | oFSH batch 19 | Ewe ID | oFSH Batch 19 | Ewe ID | oFSH batch 19 |
| 001 | 16 | 002 | 17 | 003 | 8 |
| 004 | 14 | | | | |
| Mean | 13.8 | | | | |
| SD | 4.0 | | | | |

* Intravaginal    sponge    (30  mg  flugestone    acetate)

## MERINO EWES

Location : Hamilton, Victoria.

Age :

General Condition : good condition, weight 50 kg

Study Design : The treated ewes were run in a flock of 40 untreated sheep on improved pasture

TABLE 12

| Number of Embryos Harvested | | | | | |
|---|---|---|---|---|---|
| Ewe ID | oFSH batch 12 | Ewe ID | oFSH Batch 12 | Ewe ID | oFSH batch 12 |
| 001 | 6 | 002 | 19 | 003 | 12 |
| 004 | 12 | 005 | 10 | 006 | 16 |
| 007 | 18 | 008 | 18 | 009 | 14 |
| 010 | 11 | 011 | 12 | 012 | 17 |
| 013 | 14 | 014 | 16 | 015 | 20 |
| 016 | 23 | 017 | 13 | 018 | 5 |
| Mean | 15.0 | | | | |
| SD | 4.7 | | | | |

## MERINO EWES

Location : Hamilton, Victoria.

Age : 2 - 3 year

General Condition : good condition, weight 45 - 50 kg

Study Design : The treated ewes were run in a flock of 15 untreated sheep on improved pasture

TABLE 13

| Number of Embryos Harvested | | | | | |
|---|---|---|---|---|---|
| Ewe ID | oFSH batch 21 | Ewe ID | oFSH Batch 21 | Ewe ID | oFSH batch 21 |
| M002 | 18 | M003 | 19 | M004 | 19 |
| M005 | 18 | M006 | 17 | M007 | 15 |
| M008 | 19 | M009 | 13 | M010 | 13 |
| M011 | 11 | M012 | 20 | M013 | 15 |
| M014 | 9 | M015 | 11 | M016 | 13 |
| Mean | 15.3 | | | | |
| SD | 3.5 | | | | |

## COMEBACK EWES

Location : Hamilton, Victoria.

Age : 3 year

General Condition : good condition, weight 50 kg

Study Design : The ofSH-treated ewes were run in a flock of 30 untreated sheep on improved pasture

TABLE 14

| Number of Embryos Harvested | | | | | |
|---|---|---|---|---|---|
| Ewe ID | oFSH batch 19 | Ewe ID | oFSH Batch 19 | Ewe ID | oFSH batch 19 |
| 001 | 18 | 002 | 10 | 003 | 16 |
| 004 | 11 | 005 | 14 | 006 | 2 |
| 007 | 15 | 008 | 13 | 009 | 26 |
| 010 | 12 | | | | |
| Mean | 13.7 | | | | |
| SD | 6.1 | | | | |

## COMEBACK EWES

Location : Hamilton, Victoria.

Age :

General Condition : light condition, weight 45 kg

Study Design : The treated ewes were run in a flock of 10 untreated sheep on improved pasture. All ewes had been treated 4 months previously.

TABLE 15

| Number of Embryos Harvested | | | | | |
|---|---|---|---|---|---|
| Ewe ID | oFSH batch 19 | Ewe ID | oFSH Batch 19 | Ewe ID | oFSH batch 19 |
| 001 | 16 | 002 | 17 | 003 | 5 |
| 004 | 20 | 005 | 12 | 006 | 2 |
| 007 | 13 | 008 | 14 | 009 | 23 |
| Mean | 13.6 | | | | |
| SD | 6.7 | | | | |

## LINCOLN SHEEP

Location : Hamilton, Victoria.

Age :

General Condition : heavy condition, weight 60 kg

Study Design : The treated ewes were run in a flock of 10 untreated sheep on improved pasture.

TABLE 16

| Number of Embryos Harvested | | | |
|---|---|---|---|
| Ewe ID | oFSH batch 19 | Ewe ID | oFSH Batch 19 |
| 001 | 11 | 002 | 9 |
| Mean | 10.0 | | |
| SD | 1.4 | | |

## EXAMPLE 10

Additional trials undertaken in goats using the simplified, standardised technique of a single intramuscular injection of 25 units of oFSH dissolved in physiological saline.

### Treatment Protocol

Day 1 - Chronogest sponge * inserted (40 mg)
Day 16 - 400 iu PMSG and single intramuscular injection of 25 units of oFSH
Day 18 - Sponge withdrawal
Day 20 - Endoscopic artificial insemination with fresh or frozen semen and/or natural mating
Day 26 - Flushing of embryos

### ANGORA GOATS

Location : Hamilton, Victoria.
Age : 4 - 7 years
General Condition : fair condition, weight 40 kg
Study Design : The treated does were run in a flock of 150 untreated does on improved pasture.

TABLE 17

| Number of Embryos Harvested | | | | | |
|---|---|---|---|---|---|
| Doe ID | oFSH batch 21 | Doe ID | oFSH Batch 21 | Doe ID | oFSH batch 21 |
| 001 | 6 | 002 | 13 | 003 | 9 |
| 004 | 9 | 005 | 4 | 006 | 6 |
| 006 | 20 | 008 | 16 | 009 | 9 |
| Mean | 10.2 | | | | |
| SD | 5.2 | | | | |

* Intravaginal sponge (40 mg flugestone acetate)

18

ANGORA GOATS

Location : Hamilton/Geelong, Victoria.
Age : 3 - 8 years
General Condition : fair to good condition, weight 35 - 50 kg
Study Design : The treated does were run in a flock of 200 untreated goats on improved pasture. Small groups of does were superovulated under varying pasture conditions over a one year period. Individual results were not kept.

TABLE 18

| Group No. | No. Does | Total No. Embroys from Group | Ave. Number Embryos/doe |
|---|---|---|---|
| 1 | 4 | 51 | 12.8 |
| 2 | 5 | 36 | 7.2 |
| 3 | 10 | 104 | 10.4 |
| 4 | 10 | 162 | 16.2 |
| 5 | 10 | 99 | 9.9 |
| 6 | 6 | 68 | 11.3 |
| 7 | 5 | 91 | 18.2 |
| 8 | 7 | 66 | 9.4 |
| 9 | 7 | 78 | 11.1 |
| 10 | 6 | 96 | 16.0 |
| 11 | 6 | 79 | 13.1 |
| Total | 76 | 930 | |
| Mean | | 12.2 | 12.3 |
| SD | | | 3.3 |

## TABLE 19

## Recommended Dosage Regimen

## of OFSH in Cattle (units NIH)

### Total Dose

| Live Weight Kg | 500 | 450 | 400 | 350 | 300 | 250 | 200 |
|---|---|---|---|---|---|---|---|
| 3 equal doses given over 8 hrs at 9am, 1pm & 5pm | 50 | 45 | 40 | 35 | 30 | 25 | 20 |

EXAMPLE 11

19

## CATTLE

Additional trials undertaken in cattle using the simplified, standardised technique of 3 equal amounts of oFSH dissolved in physiological saline given over 1 day at 9am, 1pm and 5pm. The dosage of oFSH was adjusted according to the liveweight of the cattle (see Table 1A).

## Treatment Protocol

(a) 2 x Prostaglandin Estrumate injections 10 - 12 days apart
(b) Begin oFSH injections 8 - 10 days after last injection of Estrumate
Inject total dosage according to liveweight of cattle as specified below as 3 equal amounts at 9am, 1 pm and 5pm by deep intramuscular injection
(c) 1 x Estrumate given at 9am on day 3 after injection of oFSH
(d) Cows artificially inseminated and/or naturally mated 5 - 6 days after oFSH treatment
(e) 8 days after mating, the treated cows are separated from the herd and the embryos flushed from their uteri using flushing media

Synchronisation of oestrus cycles should be established using injections of prostaglandin F2, a prostaglandin F2 analogue or any other means found satisfactory.

Treatment of cattle with oFSH should begin 8 to 10 days after oestrus.

## JERSEY-HEREFORD CROSS COWS

Location : Armidale, NSW
Age : 3 years
General Condition : weight 250 kg
Study Design : The treated cows were run in a herd of 15 untreated cows on improved pasture.

The dosage regimen of oFSH was 25u per cow.

TABLE 20

| Number of Embryos Harvested | | | | | |
|---|---|---|---|---|---|
| Cow ID | oFSH batch 9 | Cow ID | oFSH Batch 9 | Cow ID | oFSH batch 9 |
| 301 | 11 | 305 | 8 | 309 | 24 |
| 313 | 18 | 317 | 11 | 321 | 14 |
| 302 | 15 | 306 | 15 | 310 | 6 |
| 314 | 6 | 318 | 13 | | |
| Mean | 12.8 | | | | |
| SD | 5.3 | | | | |

## HEREFORD COWS

Location : Armidale, NSW
Age : 2 years

General Condition : Good, weight 250 kg
Study Design : The treated cows were run in a herd of 30 untreated cows on improved pasture.

The dosage regimen of oFSH was 25u per cow.

TABLE 21

| Number of Embryos Harvested | | | | | |
|---|---|---|---|---|---|
| Cow ID | oFSH batch 19 | Cow ID | oFSH Batch 19 | Cow ID | oFSH batch 19 |
| 13 | 3 | 272 | 11 | 62 | 11 |
| 80 | 18 | 129 | 20 | 141 | 8 |
| 143 | 17 | 145 | 5 | 147 | 14 |
| Mean | 11.9 | | | | |
| SD | 5.9 | | | | |

HEREFORD COWS

Location : Armidale, NSW
Age : 2 years
General Condition : Very good, weight 350 kg
Study Design : The treated cows were run in a herd of 20 untreated cows on improved pasture.

The dosage regimen of oFSH was 35u per cow.

TABLE 22

| Number of Embryos Harvested | | | | | |
|---|---|---|---|---|---|
| Cow ID | oFSH batch 24 | Cow ID | oFSH Batch 24 | Cow ID | oFSH batch 24 |
| 42 | 8 | 89 | 14 | 100 | 14 |
| 137 | 15 | 252 | 12 | 254 | 11 |
| 256 | 5 | | | | |
| Mean | 11.3 | | | | |
| SD | 3.6 | | | | |

SIMMENTAL COWS

Location : Hamilton, Victoria
Age : 4 - 5 years
General Condition : weight 500 - 600 kg
Study Design : The treated cows were run in a herd of 50 untreated cows on improved pasture.

The dosage regimen of oFSH was 50u per cow.

21

TABLE 23

| Number of Embryos Harvested | | | | | |
|---|---|---|---|---|---|
| Cow ID | oFSH batch | Cow ID | oFSH Batch | Cow ID | oFSH batch |
| 311 | 16 | Eleanor | 21 | Blazey | 12 |
| 341 | 12 | 452 | 10 | 348 | 14 |
| 376 | 8 | | | | |
| Mean | 13.3 | | | | |
| SD | 4.3 | | | | |

## SIMMENTAL COWS

Location : Hamilton, Victoria
Age : 4 - 5 years
General Condition : very fat, weight 550 - 650 kg
Study Design : The treated cows were run as a group on improved pasture.

The dosage regimen of oFSH was 50u per cow.

TABLE 24

| Number of Embryos Harvested | | | | | |
|---|---|---|---|---|---|
| Cow ID | oFSH batch | Cow ID | oFSH Batch | Cow ID | oFSH batch |
| 001 | 12 | 002 | 6 | 003 | 10 |
| 004 | 8 | 005 | 10 | | |
| Mean | 9.2 | | | | |
| SD | 2.3 | | | | |

Finally, it is to be understood that various other modifications and/or alterations may be made without departing from the spirit of the present invention as outlined herein.

## Claims

1. A method of increasing the number of ova produced during ovulation in female animals which method includes administering to the animal an ovine follicle stimulating hormone (as hereinbefore defined) wherein when the animal is a bovine animal the hormone is administered in a total amount of from approximately 20 to 60 u in at least three substantially equal doses at at least three regular intervals during a period of 1 day, dependant upon the size of the animal; and
when the animal is an ovine or caprine animal the hormone is administered in an amount of from approximately 20 to 40 u in a single dose.

2. A method according to claim 1 wherein the ovine follicle-stimulating hormone, includes a synthetic follicle-stimulating hormone derivative thereof or bioprecursor therefor formed from the cloning of the gene coding for ovine follicle-stimulating hormone or fragment thereof and expression in a prokaryotic or eukaryotic organism.

22

3. A method according to claim 2 wherein the animal is a sheep or goat and ovine follicle-stimulating hormone is administered in an amount of approximately 20 to 30 u in a single dose.

4. A method according to claim 2 wherein the animal is bovine and ovine follicle-stimulating hormone is administered in three substantially equal amounts totalling approximately 0.06 to 0.24 u/kg live weight in a single day.

5. A method according to claim 4 wherein the total dose rate is approximately 0.09 to 0.15 u/kg live weight in a single day.

6. A method according to claim 1 further including the preliminary step of administering to the animal an effective amount of a synchronising agent at a preselected interval of time prior to the initiation of the ruminant follicle-stimulating hormone treatment.

7. A method according to claim 6 wherein approximately 250 ug to approximately 100 mg of a synchronising agent is administered to the animal approximately 8 to 15 days prior to initiation of ruminant follicle-stimulating hormone treatment.

8. A method according to claim 1 further including the step of administering to the animal an effective amount of a luteinizing agent before, during or after the ruminant follicle-stimulating hormone treatment.

9. A method according to claim 8 wherein the luteinizing agent is a prostaglandin or prostaglandin analogue and is administered in an amount of approximately 250 ug to approximately 1000 ug approximately 1 to 3 days after the initiation of ruminant follicle-stimulating hormone treatment.

10. A method according to claim 9 further including the step of administering to the animal an effective amount of a gonadotrophin or derivative thereof during or before the ruminant follicle-stimulating hormone treatment.

11. A method according to claim 10 wherein gonadotrophin is pregnant mare serum gonadotrophin and approximately 100 i.u. to 1000 i.u. of gonadotrophin is administered to the animal during the first day of ruminant follicle-stimulating hormone treatment.

12. A veterinary composition including an effective amount of an ovine follicle-stimulating hormone, (as hereinbefore defined) and an effective amount of a veterinarily acceptable carrier or excipient therefor, for use in the method according to claim 1.

13. A veterinary composition according to claim 12 wherein the veterinary composition is in an injectable form and includes an effective amount of ovine follicle-stimulating hormone in a lyophilised form and an effective amount of an aqueous solvent therefor.

14. A kit of parts including a supply of an ovine follicle-stimulating hormone (as hereinbefore defined) in a lyophilised form in a suitable container; and a supply of an aqueous solvent therefor in a suitable container for use in a method according to claim 1.

15. A kit of parts according to claim 14 further including a supply of a synchronising agent in a suitable container.

16. A kit of parts according to claim 15 further including a supply of a luteinising agent in a suitable container.

17. A kit of parts according to claim 16 further including a supply of a gonadotrophin or derivative thereof in a suitable container.